# EUROPÄISCHE PATENTSCHRIFT

(11) **EP 0 427 071 B1**
(45) Veröffentlichungstag und Bekanntmachung des Hinweises auf die Patenterteilung: **02.03.1994**
(21) Anmeldenummer: 90120648.2
(22) Anmeldetag: 27.10.1990
(51) Int. Cl.: C07C 303/02, C07C 309/46, C07C 309/48

(54) **Verfahren zur Herstellung von 2-Aminobenzol-disulfonsäuren-(1,4)**
Process for the preparation of 2-aminobenzene-1,4-disulfonic acids
Procédé pour la préparation d'acides 2-aminobenzène-1,4-disulfoniques

(30) Priorität: 09.11.1989 DE 3937342
(43) Veröffentlichungstag der Anmeldung: 15.05.1991
(73) Patentinhaber: BAYER AG, 51368 Leverkusen (DE)
(72) Erfinder: Schimpf, Rolf, Dr., W-5090 Leverkusen 1 (DE); Horstmann, Walter, Dr., W-5060 Bergisch Gladbach 2 (DE)

(56) Entgegenhaltungen:
- CH-A- 391 724
- DE-A- 2 534 176
- DE-B- 1 131 223
- DE-C- 77 192
- GB-A- 285 488
- US-A- 2 965 675

## Beschreibung

Die vorliegende Erfindung betrifft ein verbessertes Verfahren zur Herstellung von 2-Aminobenzol-disulfonsäuren-(1,4) und die neue Verbindung 6-Chlor-2-aminobenzol-disulfonsäure-(1,4).

Zur Herstellung von 2-Aminobenzol-disulfonsäuren-(1,4) sind verschiedene Verfahren bekannt. Man kann z.B. Anilinsulfonsäure-(3) mit Oleum 6 Stunden lang auf 160°C erhitzen, das sich dabei ergebende Reaktionsgemisch mit Wasser verdünnen und 5 Stunden unter Rückfluß kochen. Beim Aussalzen mit Natriumchlorid erhält man 2-Aminobenzol-disulfonsäure-(1,4) in 90 %iger Ausbeute (siehe Friedländer 16, 382). Dieses Verfahren weist jedoch eine Reihe von Nachteilen auf: So muß das Ausgangsmaterial Anilinsulfonsäure-(3) erst getrocknet werden, bevor es eingesetzt werden kann. Die Umsetzung mit Oleum bei 160°C erfordert besonderen technischen Aufwand und Sicherheitsmaßnahmen. Die anfallende salzhaltige Dünnsäure ist nur schwierig aufzuarbeiten, weil bei der Wiedergewinnung von Schwefeldioxid infolge von Salzablagerungen erhebliche Probleme auftreten.

Anstelle von Oleum kann man auch Chlorsulfonsäure einsetzen (siehe Ber. 21, 3412 (1888)). Dabei treten die gleichen Nachteile auf wie bei der Verwendung von Oleum.

Bei einem anderen Verfahren zur Herstellung von 2-Aminobenzol-disulfonsäure-(1,4) wird 2-Nitrobenzol-disulfonsäure-(1,4) mit Eisen in essigsaurer Lösung reduziert (siehe Friedländer 4, 38). Die als Ausgangsprodukt benötigte 2-Nitrobenzol-disulfonsäure-(1,4) wird dabei durch Umsetzung von 4-Chlor-3-nitrobenzol-sulfonsäure-(1) mit 1,2 Mol Natriumsulfit in alkalischer Lösung bei einem pH-Wert von 8 bis 11 und Temperaturen von 100°C hergestellt. Die Nacharbeitung dieses Verfahrens führte zu Ausbeuten von maximal 76 % der Theorie, bezogen auf eingesetzte 4-Chlor-3-nitrobenzol-sulfonsäure-(1) (siehe Beispiel 3). Neben der mäßigen Ausbeute hat dieses Verfahren weitere Nachteile: So wird die 2-Nitrobenzol-disulfonsäure-(1,4) mit Kochsalz ausgesalzen und zwischenisoliert. Die nach der Reduktion mit Eisen vorliegende Lösung fällt in großer Verdünnung an und muß erst aufkonzentriert werden, bevor 2-Aminobenzol-disulfonsäure-(1,4) abgetrennt werden kann.

Die Reduktion von 2-Nitrobenzol-disulfonsäure-(1,4) mit Eisen wurde auch als halbkontinuierliches Verfahren beschrieben (siehe DE-OS 25 34 176). Dabei wurde eine Ausbeute von 74 % erzielt.

Es ist ferner bekannt, daß bei der Umsetzung von Chlornitrobenzol-sulfonsäuren mit Sulfiten Nebenreaktionen auftreten können. Neben dem gewünschten Austausch des Chloratoms gegen die Sulfogruppe kann die Nitrogruppe reduziert werden (Piria-Reaktion) und/oder das Halogenatom hydrolytisch abgespalten werden. Im letzteren Fall entsteht aus 4-Chlor-3-nitrobenzol-sulfonsäure-(1) die 2-Aminophenol-4-sulfonsäure (Houben-Weyl, Methoden der organischen Chemie, Band 11/1 (1957), Seite 457).

Es wurde nun ein Verfahren zur Herstellung von 2-Aminobenzol-disulfonsäuren-(1,4) der Formel (I) gefunden, 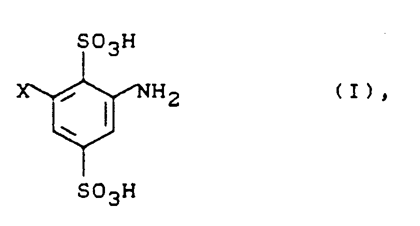
in der
- X: für Wasserstoff oder Chlor steht,
bei dem man 4-Chlor-3-nitrobenzol-sulfonsäuren der Formel (II) 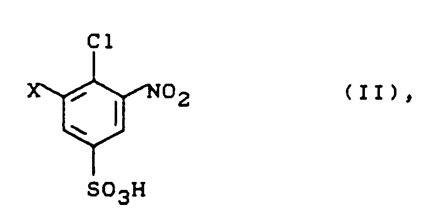
in der
- X: für Wasserstoff oder Chlor steht,
in wäßrig alkalischem Milieu mit Natriumsulfit umsetzt und anschließend mit Eisen reduziert, das dadurch gekennzeichnet ist, daß man die Umsetzung mit Natriumsulfit bei 40 bis 90°C durchführt, vor der Reduktion mit Eisen keine Zwischenisolierung vornimmt und nach dem Abfiltrieren des Eisenoxids 2-Aminobenzol-disulfonsäuren-(1,4) der Formel (I) isoliert, nachdem man im Filtrat bei 70 bis 90°C mit Mineralsäure einen pH-Wert von < 1 eingestellt und danach abgekühlt hat.

4-Chlor-3-nitrobenzol-sulfonsäure (Formel (II), X = Wasserstoff) ist ein bekanntes Produkt (siehe P. Fischer, Ber. 24, 3187-3188 (1891)), das auch bei den Verfahren des Standes der Technik zum Einsatz kommt. 4,5-Dichlor-3-nitrobenzol-sulfonsäure (Formel (II), X = Chlor) kann z.B. analog zu 4-Chlor-3-nitrobenzol-sulfonsäure hergestellt werden.

Erfindungsgemäß wird die Umsetzung mit Natriumsulfit bei 40 bis 90°C, vorzugsweise 60 bis 80°C durchgeführt. Man kann dabei z.B. so vorgehen, daß man 1 Mol einer 4-Chlor-3-nitrobenzol-sulfonsäure der Formel (II) mit 1,0 bis 1,5 Mol, vorzugsweise 1,1 bis 1,3 Mol Natriumsulfit oder einer äquimolaren Menge einer äquimolaren Mischung aus Natriumhydrogensulfit und Natronlauge zusammen mit Wasser bei einem pH-Wert von 8 bis 12, vorzugsweise 10 bis 11,5, 1 bis 3 Stunden auf die Umsetzungstemperatur erhitzt. Der wesentliche Unterschied zum Stand der Technik ist bei dieser Verfahrensstufe die niedrigere Reaktionstemperatur.

Die nach der ersten Reaktionsstufe im Reaktionsgemisch vorliegende 2-Nitrobenzol-disulfonsäure-(1,4) wird im Gegensatz zum Stand der Technik beim erfindungsgemäßen Verfahren nicht isoliert.

Die Reduktion mit Eisen kann z.B. so erfolgen, daß man das nach der Umsetzung mit Natriumsulfit erhaltene Gemisch, gegebenenfalls nach Erniedrigung des pH-Wertes auf 5 bis 7 (z.B. durch Zusatz von Essigsäure), in eine vorgelegte Mischung aus Wasser und Eisen, das mit Essigsäure angeätzt worden ist, einlaufen läßt. Geeignete Temperaturen für die Reduktion sind z.B. 85 bis 100°C. Nach beendeter Reduktion stellt man das Reduktionsgemisch alkalisch, z.B. durch Zugabe von Soda, und trennt das gebildete Eisenoxid ab, z.B. durch Filtration. Es ist vorteilhaft, das dabei anfallende Filtrat gemeinsam mit dem Filtrat aufzuarbeiten, das man erhält, wenn man das abgetrennte Eisenoxid mit warmem Wasser auswäscht.

Im Gegensatz zum Stand der Technik werden so hergestellte 2-Aminobenzol-disulfonsäuren-(1,4) der Formel (I) nicht durch Einengung der vereinigten Filtrate isoliert. Erfindungsgemäß werden die vereinigten Filtrate zunächst auf eine Temperatur von 70 bis 90°C gebracht, mit Mineralsäure, vorzugsweise Schwefelsäure, ein pH-Wert von < 1 eingestellt und danach abgekühlt, z.B. auf 10 bis 30°C. Dabei fällt die hergestellte 2-Aminobenzoldisulfonsäure-(1,4) der Formel (I) in Form von Kristallen aus, die z.B. durch Filtration abgetrennt werden können.

2-Aminobenzol-disulfonsäure-(1,4) ist ein Zwischenprodukt zur Herstellung von Azofarbstoffen (siehe DE-PS en 416 617, 450 998 und 453 133) und optischen Aufhellern (siehe DE-OS 24 03 455).

Es wurde weiterhin gefunden, daß sich bei der erfindungsgemäßen Herstellung von 2-Aminobenzol-disulfonsäuren-(1,4) der Formel (I) bei der Umsetzung mit Natriumsulfit und bei der Reduktion mit Eisen neue 2-N-Sulfonaminobenzol-disulfonsäuren-(1,4) der Formel (III) bilden 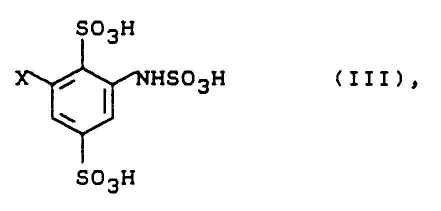
in der
- X: für Wasserstoff oder Chlor steht,
die sich bei der erfindungsgemäßen Isolierung der 2-Aminobenzol-disulfonsäuren-(1,4) der Formel (I) in diese umwandeln und so eine deutlich höhere Ausbeute ergeben. Diese beträgt im allgemeinen 88 bis 92 % der Theorie.

Das erfindungsgemäße Verfahren zeichnet sich außerdem durch eine einfache Arbeitsweise aus, die keine besonderen Sicherheitsvorkehrungen erfordert.

Im Hinblick auf den eingangs geschilderten Stand der Technik sind die mit dem erfindungsgemäßen Verfahren erzielbaren Vorteile als außerordentlich überraschend anzusehen.

Die Verbindungen der Formel (III) können aus Gemischen von 2-Aminobenzol-disulfonsäure-(1,4) und 2-N-Sulfonaminobenzol-disulfonsäure-(1,4) bzw. 2-Amino-6-chlorbenzol-disulfonsäure-(1,4) und 2-N-Sulfonamino-6-chlorbenzol-disulfonsäure-(1,4), wie sie beim erfindungsgemäßen Verfahren zur Herstellung von 2-Aminobenzol-disulfonsäuren-(1,4) der Formel (I) nach der Reduktion mit Eisen anfallen, isoliert werden, z.B. indem man das nach der Abtrennung des Eisens vorliegende Filtrat einengt, den ausfallenden Niederschlag abfiltriert und mehrmals, z.B. 2- bis 4-mal, aus einem Methanol-Wasser-Gemisch umkristalliert. Reine Produkte der Formel (III) können erhalten werden, indem man anschließend an die Umkristallisationen eine präparative Dünnschichtchromatographie durchführt. Untersuchungen mittels ¹H-NMR-Spektroskopie an auf diese Weise isolierten und gereinigten Verbindungen der Formel (III) bestätigen die in Formel (III) angegebene Struktur.

### Beispiele

### Beispiel 1

484 g feuchtes Natriumsalz der 4-Chlor-3-nitrobenzolsulfonsäure (mit anhaftender Schwefelsäure und Natriumhydrogensulfat), entsprechend 1 Mol, wurden mit 627 ml Wasser vorgelegt und mit 63 ml 50 gew.-%iger Natronlauge auf einen pH-Wert von 10,5 eingestellt. Dann wurden 145 g wasserfreies Natriumsulfit (99 gew.-%ig, 1,16 Mol) hinzugegeben und das Reaktionsgemisch auf 60°C erwärmt. Nach 1,5 stündigem Rühren bei 60°C wurde durch Zugabe von Essigsäure ein pH-Wert von 6 bis 7 eingestellt. Das so erhaltene Gemisch wurde dann innerhalb einer Stunde bei 98°C in ein vorgelegtes Gemisch aus 414 g Eisenspänen (7,4 Mol), 150 ml Wasser und 2 ml Essigsäure eingetropft. 20 Minuten nach Ende der Zugabe wurde das Reaktionsgemisch mit Soda alkalisch eingestellt und das ausfallende Eisenoxid abgesaugt. Das Eisenoxid wurde anschließend mit 300 ml warmem Wasser abgewaschen. Die beiden Filtrate wurden vereinigt, auf 80°C erwärmt und mit 132 ml 78 gew.-%iger Schwefelsäure (1,8 Mol) angesäuert, wobei sich ein pH-Wert von 0,5 ergab. Nach 30 minütigem Rühren bei 80°C wurde das Gemisch unter Rühren auf 20°C abgekühlt und die dabei ausfallende weiße, grob kristalline 2-Aminobenzol-disulfonsäure-(1,4) abfiltriert Es wurden 342,7 g feuchtes Produkt erhalten, was 231 g 100 %igem Produkt mit einer Ausbeute von 91 % der Theorie entsprach.

### Beispiel 2

443 g feuchtes Natriumsalz der 4,5-Dichlor-3-nitrobenzol-sulfonsäure-(1) (mit anhaftender Schwefelsäure und Natriumhydrogensulfat), entsprechend 1 Mol, wurden in 400 ml Wasser vorgelegt und mit konzentrierter Natronlauge auf einen pH-Wert von 11 eingestellt. Dann wurden 299 ml Natriumhydrogensulfitlösung (enthaltend 435 g Natriumhydrogensulfit pro Liter = 1,25 Mol) und 66 ml konzentrierte Natronlauge (1,26 Mol) simultan so zulaufen gelassen, daß der pH-Wert im Bereich von 8 bis 11 lag und die Temperatur 35°C nicht überschritt. Anschließend wurde das Reaktionsgemisch auf 80°C erhitzt und 30 Minuten bei dieser Temperatur nachgerührt. Dann wurde die Suspension durch Zugabe von 6 ml Essigsäure auf einen pH-Wert im Bereich 6 bis 7 abgestumpft. Innerhalb einer Stunde wurde dieses Reaktionsgemisch bei 98°C in ein vorgelegtes Gemisch aus 414 g Eisenspänen (7,4 Mol), 150 ml Wasser und 2 ml Essigsäure eingetropft. Nach Ende der Zugabe wurde noch 20 Minuten nachgerührt, dann wurde das Reaktionsgemisch mit Soda auf einen pH-Wert von 8 bis 9 gebracht und das ausgefallene Eisenoxid abgesaugt. Das Eisenoxid wurde anschließend mit 300 ml warmem Wasser ausgewaschen und die beiden Filtrate vereinigt. Die vereinigten Filtrate wurden auf 80°C erwärmt, mit 84 ml 78 gew.-%iger Schwefelsäure (1,14 Mol) angesäuert, was einen pH-Wert von 0,5 ergab, und noch 30 Minuten bei 80°C nachgerührt. Dann wurde das Gemisch unter Rühren auf 20°C abgekühlt und die ausfallende gelblich gefärbte, grob kristalline 6-Chlor-2-aminobenzol-disulfonsäure-(1,4) abfiltriert. Es wurden 352,9 g feuchtes Produkt erhalten, was 253 g 100 %igem Produkt und einer Ausbeute von 88 % der Theorie entsprach.

Die neue 6-Chlor-2-aminobenzol-disulfonsäure-(1,4) wurde durch Elementaranalyse und das ¹H-NMR-Spektrum in NaOD als Lösungsmittel charakterisiert (δ 7,2 ppm-Singulett).

### Beispiel 3 (Nachbearbeitung von Friedländer 4, 38)

484 g feuchtes Natriumsalz der 4-Chlor-3-nitrobenzolsulfonsäure-(1) (mit anhaftender Schwefelsäure und Natriumhydrogensulfat), entsprechend 1 Mol, wurden in 650 ml Wasser vorgelegt und mit 59 ml 50 gew.-%iger Natronlauge auf einen pH-Wert von 7 bis 8 eingestellt. Dann wurden 147 g wasserfreies Natriumsulfit (1,2 Mol) hinzugegeben, das Reaktionsgemisch auf Siedetemperatur erhitzt und 2 Stunden bei dieser Temperatur belassen. Danach wurden 115 g Natriumchlorid (1,97 Mol) zugefügt, unter Rühren auf 24°C abgekühlt und der ausfallende Niederschlag abfiltriert. Der isolierte Feststoff wurde in 2.300 ml Wasser aufgelöst und diese Lösung im Verlauf einer Stunde bei 98 °C in ein vorgelegtes Gemisch aus 414 g Eisen (7,4 Mol), 150 ml Wasser und 2 ml Essigsäure zugetropft. 20 Minuten nach Ende der Zugabe wurde das Reaktionsgemisch mit Soda alkalisch gestellt und das ausgefallene Eisenoxid abgesaugt. Danach wurde das Eisenoxid mit 300 ml warmem Wasser ausgewaschen. Die vereinigten Filtrate wurden auf 80°C erwärmt, mit 182 ml 78 gew.-%iger Schwefelsäure (2,47 Mol) angesäuert, was einen pH-Wert von 0,5 ergab. Es wurden noch 30 Minuten bei 80°C nachgerührt, dann wurden bei 102 bis 108°C bis zur beginnenden Kristallisation 1.600 ml Wasser abdestilliert. Nach dem Abkühlen auf 20°C unter Rühren wurde der ausgefallene Niederschlag abfiltriert. Es wurden 235 g feuchte 2-Aminobenzol-disulfonsäure-(1,4) in Form von hellgrauen, groben Kristallen erhalten. Das entspricht 192,2 g 100 %igem Produkt und einer Ausbeute von 76 % der Theorie.

## Patentansprüche

1. Verfahren zur Herstellung von 2-Aminobenzol-disulfonsäuren-(1,4) der Formel (I) 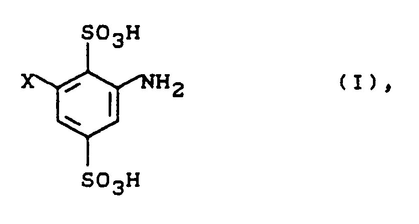 in der
X für Wasserstoff oder Chlor steht,
bei dem man 4-Chlor-3-nitrobenzol-sulfonsäuren der Formel (II) 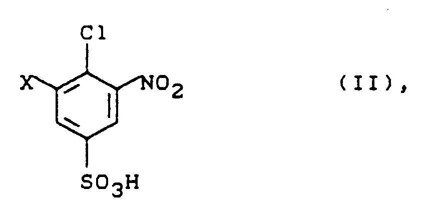 in der
X für Wasserstoff oder Chlor steht,
in wäßrig alkalischem Milieu mit Natriumsulfit umsetzt und anschließend mit Eisen reduziert, dadurch gekennzeichnet, daß man die Umsetzung mit Natriumsulfit bei 40 bis 90°C durchführt, vor der Reduktion mit Eisen keine Zwischenisolierung vornimmt und nach dem Abfiltrieren des Eisenoxids 2-Aminobenzol-disulfonsäuren-(1,4) der Formel (I) isoliert, nach dem man im Filtrat bei 70 bis 90°C mit Mineralsäure einen pH-Wert von < 1 eingestellt und danach abgekühlt hat.

2. Verfahren nach Anspruch 1, dadurch gekennzeichnet, daß man 1,0 bis 1,5 Mol Natriumsulfit pro Mol der Verbindung der Formel (II) einsetzt.

3. Verfahren nach Ansprüchen 1 und 2, dadurch gekennzeichnet, daß man die Umsetzung mit Natriumsulfit bei 60 bis 80°C durchführt.

4. Verfahren nach Ansprüchen 1 bis 3, dadurch gekennzeichnet, daß man die Umsetzung mit Natriumsulfit bei einem pH-Wert von 8 bis 12 durchführt.

5. Verfahren nach Ansprüchen 1 bis 4, dadurch gekennzeichnet, daß man die Reduktion mit Eisen bei 85 bis 100°C und bei einem pH-Wert im Bereich von 5 bis 7 durchführt.

6. Verfahren nach Ansprüchen 1 bis 5, dadurch gekennzeichnet, daß man als Mineralsäure Schwefelsäure verwendet.

7. Verfahren nach Ansprüchen 1 bis 6, dadurch gekennzeichnet, daß man abschließend auf eine Temperatur von 10 bis 30°C abkühlt.

## Claims

1. Process for the preparation of 2-aminobenzene-1,4-disulphonic acids of the formula (I) 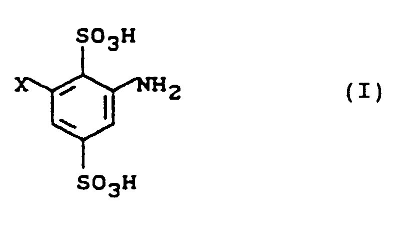 in which
X represents hydrogen or chlorine,
in which 4-chloro-3-nitrobenzene-sulphonic acids of the formula (II) 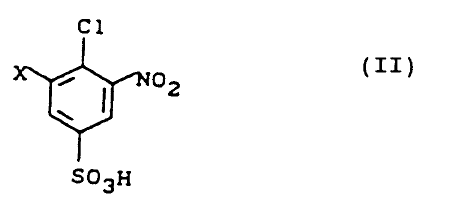 in which
X represents hydrogen or chlorine,
are reacted with sodium sulphite in an aqueous alkaline medium and then reduced with iron, characterized in that the reaction with sodium sulphite is carried out at 40 to 90°C, no intermediate isolation is carried out before the reduction with iron and 2-aminobenzene-1,4-disulphonic acids of the formula (I) are isolated after filtering off the iron oxide, after a pH of < 1 has been established in the filtrate at 70 to 90°C using mineral acid and after the filtrate has been cooled.

2. Process according to Claim 1, characterized in that 1.0 to 1.5 moles of sodium sulphite are employed per mole of the compound of the formula (II).

3. Process according to Claims 1 and 2, characterized in that the reaction with sodium sulphite is carried out at 60 to 80°C.

4. Process according to Claims 1 to 3, characterized in that the reaction with sodium sulphite is carried out at a pH of 8 to 12.

5. Process according to Claims 1 to 4, characterized in that the reduction with iron is carried out at 85 to 100°C and at a pH in the range from 5 to 7.

6. Process according to Claims 1 to 5, characterized in that sulphuric acid is used as the mineral acid.

7. Process according to Claims 1 to 6, characterized in that the filtrate is finally cooled to a temperature of 10 to 30°C.

## Revendications

1. Procédé de production d'acides 2-aminobenzène-1,4-disulfoniques de formule (I) 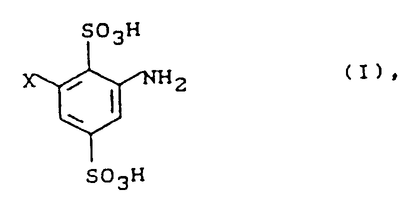 dans laquelle
X représente l'hydrogène ou le chlore,
dans lequel on fait réagir des acides 4-chloro-3-nitrobenzène-sulfoniques de formule (II) 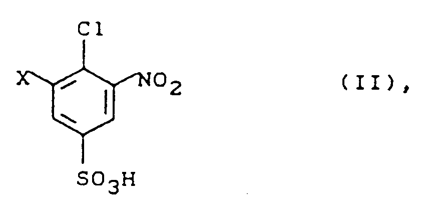 dans laquelle
X représente l'hydrogène ou le chlore,
en milieu alcalin aqueux, avec du sulfite de sodium puis on effectue une réduction avec du fer, caractérisé en ce qu'on conduit la réaction avec le sulfite de sodium à 40-90°C, on n'effectue pas d'isolement intermédiaire avant la réduction avec le fer et après séparation de l'oxyde de fer par filtration, on isole les acides 2-aminobenzène-1,4-disulfonique de formule (I), après avoir ajusté le pH du filtrat à une valeur inférieure à 1 avec un acide minéral à 70-90°C et après avoir refroidi.

2. Procédé suivant la revendication 1, caractérisé en ce qu'on utilise 1,0 à 1,5 mole de sulfite de sodium par mole de composé de formule (II).

3. Procédé suivant les revendications 1 et 2, caractérisé en ce qu'on conduit la réaction avec le sulfite de sodium à 60-80°C.

4. Procédé suivant les revendications 1 à 3, caractérisé en ce qu'on conduit la réaction avec le sulfite de sodium à un pH de 8 à 12.

5. Procédé suivant les revendications 1 à 4, caractérisé en ce qu'on conduit la réduction avec du fer à 85-100°C et à un pH de valeur comprise dans la plage de 5 à 7.

6. Procédé suivant les revendications 1 à 5, caractérisé en ce qu'on utilise comme acide minéral l'acide sulfurique.

7. Procédé suivant les revendications 1 à 6, caractérisé en ce qu'on refroidit finalement à une température de 10 à 30°C.
